(19) Europäisches Patentamt
European Patent Office
Office européen des brevets

(11) Veröffentlichungsnummer: **0 380 985 B1**

(12) # EUROPÄISCHE PATENTSCHRIFT

(45) Veröffentlichungstag der Patentschrift: **08.12.93**

(51) Int. Cl.[5]: **C07D 233/90**, A01N 43/50

(21) Anmeldenummer: **90101124.7**

(22) Anmeldetag: **20.01.90**

Die Akte enthält technische Angaben, die nach dem Eingang der Anmeldung eingereicht wurden und die nicht in dieser Patentschrift enthalten sind.

(54) **4-Aryl-2-halogenimidazol-5-carbonsäureester, ihre Herstellung und Verwendung.**

(30) Priorität: **31.01.89 DE 3902772**

(43) Veröffentlichungstag der Anmeldung: **08.08.90 Patentblatt 90/32**

(45) Bekanntmachung des Hinweises auf die Patenterteilung: **08.12.93 Patentblatt 93/49**

(84) Benannte Vertragsstaaten: **BE CH DE FR GB IT LI NL**

(56) Entgegenhaltungen:
**EP-A- 0 127 446**
**EP-A- 0 180 787**
**EP-A- 0 264 577**

(73) Patentinhaber: **BASF Aktiengesellschaft**
**Carl-Bosch-Strasse 38**
**D-67063 Ludwigshafen(DE)**

(72) Erfinder: **Wriede, Ulrich, Dr.**
**Birkenstrasse 7**
**D-6704 Mutterstadt(DE)**
Erfinder: **Hamprecht, Gerhard, Dr.**
**Rote-Turm-Strasse 28**
**D-6940 Weinheim(DE)**
Erfinder: **Koehler, Hermann, Dr.**
**In den Obstgaerten 7**
**D-6719 Bobenheim(DE)**
Erfinder: **Kuekenhoehner, Thomas, Dr.**
**Seidelstrasse 2**
**D-66710 Frankenthal(DE)**
Erfinder: **Wuerzer, Bruno, Dr.**
**Ruedigerstrasse 13**
**D-6701 Otterstadt(DE)**
Erfinder: **Westphalen, Karl-Otto, Dr.**
**Mausbergweg 58**
**D-6720 Speyer(DE)**

**EP 0 380 985 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft 4-Aryl-2-halogenimidazol-5-carbonsäureester der allgemeinen Formeln Ia und Ib

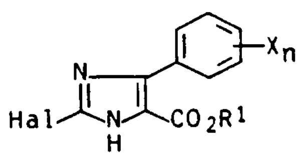

Ia

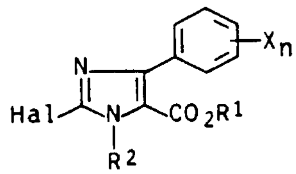

Ib

worin die Substituenten und Indizes die folgenden Bedeutungen haben:

- $R^1$ eine $C_1$-$C_6$-Alkylgruppe, eine $C_3$-$C_6$-Alkenylgruppe oder eine $C_3$-$C_6$-Alkinylgruppe
- $R^2$ eine $C_1$-$C_6$-Alkylgruppe, eine $C_3$-$C_6$-Alkenylgruppe oder eine $C_3$-$C_6$-Alkinylgruppe,
- Hal Chlor oder Brom
- n 0 bis 3
- X eine $C_1$-$C_4$-Alkylgruppe, eine $C_1$-$C_4$-Halogenalkylgruppe, eine $C_1$-$C_4$-Alkoxygruppe, eine $C_1$-$C_4$-Halogenalkoxygruppe, eine $C_1$-$C_4$-Alkylthiogruppe, eine $C_1$-$C_4$-Alkylsulfinylgruppe, eine $C_1$-$C_4$-Alkylsulfonylgruppe, eine $C_1$-$C_4$-Halogenalkylthiogruppe, eine Cyanogruppe, eine Nitrogruppe, eine Carbo-$C_1$-$C_4$-alkoxygruppe, eine N,N-Di-$C_1$-$C_4$-alkylcarbamidogruppe und/oder ein Halogenatom.

Außerdem betrifft die Erfindung Verfahren zur Herstellung der Verbindungen Ia und Ib sowie herbizide Mittel, die Cyclohexenonderivate als herbizide Wirkstoffe und Arylhalogenimidazolderivate als Antidots enthalten, sowie Verfahren zur selektiven Bekämpfung von unerwünschtem Pflanzenwuchs mit diesen herbiziden Mitteln.

Aus EP-A 174 562 sind 1-Aryltriazolcarbonsäurederivate mit pflanzenschützender Wirkung bekannt. Daneben sind 1-Arylimidazolcarbonsäureester mit wachstumsregulierenden Eigenschaften (EP-A 243 615, EP-A 264 577) und 2-Halogenimidazolcarbonsäureester mit herbizider Wirkung beschrieben (EP-A 127 446; EP-A 180 787; US-A 4.711.962; USA 4.591.377 und US-A 4.578.106).

Herbizide Wirkstoffe aus der Gruppe der Cyclohexenonderivate der Formel X,

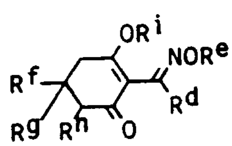

X

in welcher die Substituenten folgende Bedeutung haben:

- $R^d$ eine $C_1$-$C_4$-Alkylgruppe;
- $R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkenylgruppe oder eine Thenylgruppe (= Thienylmethylgruppe), welche durch ein Halogenatom substituiert sein kann;
- $R^f$ eine $C_1$-$C_4$-Alkylgruppe, welche einfach durch $C_1$-$C_4$-Alkylthio oder $C_1$-$C_4$-Alkoxy substituiert sein kann;

  ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, und dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;

  einen 10-gliedrigen gesättigten oder einfach ungesättigten Heterocyclus, welcher zwei Sauerstoffatome oder Schwefelatome enthält und durch bis zu drei $C_1$-$C_4$-Alkylgruppen und/oder Methoxygruppen substituiert sein kann;

  eine Phenylgruppe, eine Pyridylgruppe oder eine Isoxazolylgruppe, wobei diese Gruppen bis zu drei der folgenden Reste tragen können: $C_1$-$C_4$-Alkyl, $C_1$-$C_4$-Halogenalkyl, $C_1$-$C_4$-Alkoxy, $C_1$-$C_4$-

Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino

$R^g$ Wasserstoff, Hydroxy oder, wenn $R^f$ die Bedeutung $C_1$-$C_6$-Alkylgruppe hat, eine $C_1$-$C_6$-Alkylgruppe;

$R^h$ Wasserstoff, eine Cyanogruppe, ein Halogenatom oder eine $C_1$-$C_4$-Alkoxycarbonylgruppe und

$R^i$ Wasserstoff oder das Äquivalent eines umweltverträglichen Kations,

sind in der Literatur als Herbizide beschrieben (z.B. EP-A 228 598, EP-A 230 235, EP-A 238 021, US-A 4 432 786, DE-A 24 39 104) und dienen vorwiegend zur Bekämpfung unerwünschter Gräser in dicotylen Kulturen und in Gräsern, die nicht zur Familie der Gramineen zählen. Je nach Struktur der Substituenten und Dosis der Anwendung können Verbindungen aus dieser Gruppe auch zur selektiven Bekämpfung von unerwünschten Gräsern in Gramineen Kulturen wie Weizen und Reis eingesetzt werden.

Der Erfindung lagen Verbindungen als Aufgabe zugrunde, welche die Nachteile bei der Verwendung der obengenannten Herbizide der Formel X beheben oder zumindest so stark verringern, daß der Ernteertrag der Kulturpflanzen nicht mehr oder nicht nennenswert herabgesetzt wird.

Entsprechend der Aufgabe wurden die eingangsdefinierten 1-Aryl- und 1-Hetarylimidazoncarbonsäureester Ia und Ib gefunden. Weiterhin wurden Verfahren zur Herstellung dieser Verbindungen Ia und Ib sowie zur gemeinsamen Anwendung dieser Verbindungen mit den Herbiziden X zur Beeinflussung unerwünschten Pflanzenwachstums gefunden. Weiterhin betrifft die Erfindung Mittel, welche die Verbindungen Ia und/oder Ib sowie Herbizide des Typs X enthalten, wobei es unerheblich ist, ob der herbizide Wirkstoff und die antidotische Verbindung gemeinsam oder getrennt formuliert und ausgebracht werden bzw. bei getrennter Ausbringung, in welcher Reihenfolge herbizider Wirkstoff und Antidot appliziert werden.

Die erfindungsgemäßen Verbindungen der Formeln Ia und Ib sind auf verschiedenen Wegen zugänglich.

So erhält man die Verbindungen der Formel Ia beispielsweise, in dem man eine Carbonylverbindung II in an sich bekannter weise analog den in J. Org. Chem. 28, 3041 (1963) beschriebenen Bedingungen ohne Isolierung der Zwischenstufen nacheinander in einer wäßrigen Säure zuerst mit einem Nitrit III zu IV nitrosiert, danach zum Aminoketoester V reduziert und diesen direkt mit einem Cyanat VI in das Arylimidazolen VII überführt, welches anschließend in Substanz oder in einem inerten organischen Losungsmittel in Gegenwart oder Abwesenheit einer Base mit einem in der organischen Chemie üblichen Halogenierungsmittel zu Ia umgesetzt wird. Die einzelnen Stufen dieser Synthese sind im folgenden Reaktionsschema wiedergegeben.

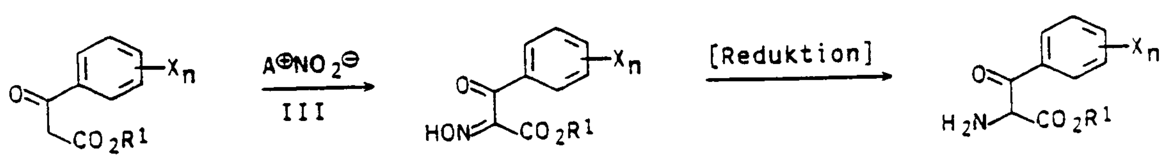


II IV V

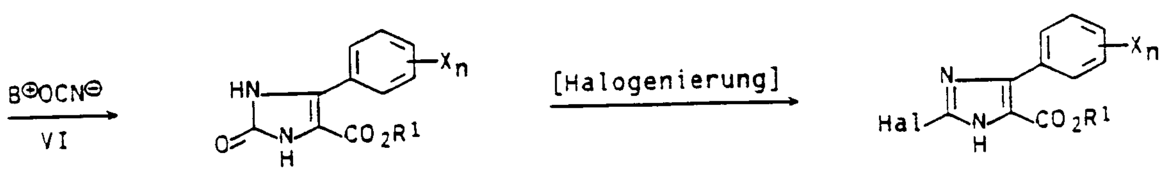


VII Ia

In der Formel III bedeutet $A^{\oplus}$ ein Alkalimetallion wie z.B. das Lithium-, das Natrium- oder das Kaliumion.

Die Nitrosierung kann unter an sich bekannten Bedingungen mit einem Nitrit III in einer wäßrigen Säure bei von -10 bis 60 °C, vorzugsweise 30 bis 40 °C kontinuierlich oder diskontinuierlich, bei Normaldruck oder unter Druck (1 bis 10 bar), durchgeführt werden.

Als Säuren kommen z.B. Carbonsäuren wie Ameisensäure, Essigsäure oder Propionsäure oder Mineralsäuren wie z.B. Salzsäure oder Schwefelsäure in Betracht, bevorzugt Essigsäure oder Schwefelsäure.

Die Isonitrosoverbindung IV kann ohne Isolierung in das Amin V überführt werden.

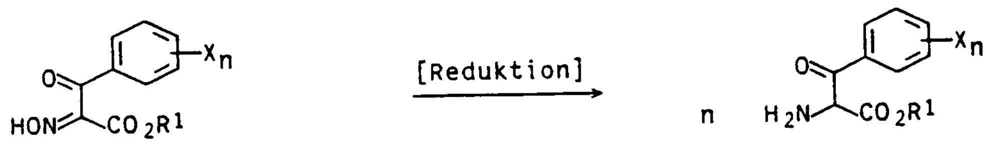


IV V

Als Reduktionsmittel dienen beispielsweise anorganische Salze wie z.B. Natriumdithionit, Natriumbisulfit, Natriumsulfit oder Zinn-II-chlorid in wäßrigen Säuren. Bevorzugt wird Natriumdithionit in den obengenannten wäßrigen Säuren.

Man kann die Verbindungen IV aber auch mit einem inerten nicht mit Wasser mischbaren organischen Lösungsmittel aus dem Reaktionsgemisch extrahieren und das isolierte Produkt in einer katalytischen Hydrierung an Edelmetall-Katalysatoren wie Platin oder Palladium oder nickelhaltigen Katalysatoren wie Raney-Nickel in einem inerten Lösungsmittel wie Essigsäure, Methanol, Ethanol oder Tetrahydrofuran bei 25 bis 50 ° C, vorzugsweise 25 ° C, unter Druck von 1 bis 100 bar, vorzugsweise 1 bis 50 bar, kontinuierlich oder diskontinuierlich mit Wasserstoff reduzieren.

Das Amin V kann ohne weitere Reinigung bei Temperaturen von 25 bis 100 ° C, mit einem Cyanat VI, worin $B^{\oplus}$ beispielsweise ein Ammonium-, Natrium-oder Kaliumion bedeutet, in den obengenannten wäßrigen Säuren mit oder ohne Zusatz eines inerten organischen Lösungsmittels wie z.B. Methanol, Ethanol oder Tetrahydrofuran zur Verbindung VII cyclisiert werden.

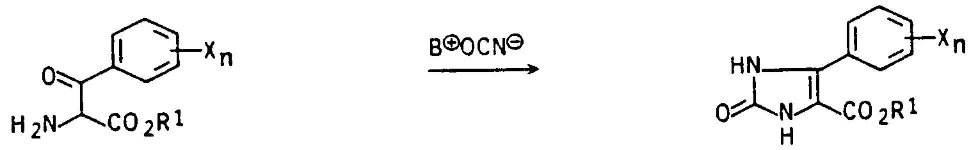


V VI VII

Die anschließende Umsetzung des Imidazols VII mit einem Halogenierungsmittel liefert Ia.

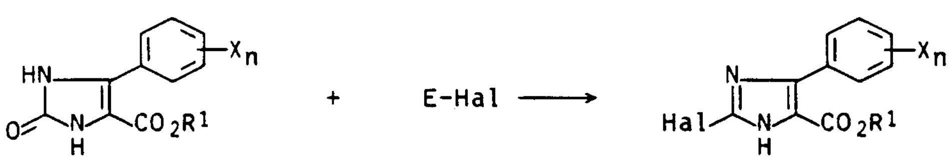


VII Ia

Geeignete Halogenierungsmittel (E-Hal) sind beispielsweise Oxyhalogenide wie z.B. Phosphoroxytrichlorid bzw.- tribromid, Thionilchlorid bzw. -bromid oder Phosgen oder Halogenide wie z.B. Phosphorpentachlorid bzw. -pentabromid, Phosphortrichlorid bzw. -tribromid oder Schwefeltetrachlorid bzw. -tetrabromid.

Bevorzugt werden Phosphoroxychlorid oder Phosphoroxybromid.

Die Reaktion kann mit oder ohne Lösungsmittel bei Temperaturen von 0 bis 180 ° C, vorzugsweise 40 bis 140 ° C, bei Normaldruck oder unter Druck (1 bis 10 bar, vorzugsweise 1 bis 3 bar), kontinuierlich oder diskontinuierlich gegebenenfalls in Anwesenheit einer Base durchgeführt werden.

Für diese Umsetzung geeignete Basen sind tertiäre Amine wie z.B. Triethylamin, Diisopropylamin, N,N-Dimethylanilin, N,N-Dimethyl-p-aminopyridin, Pyridin, Isochinolin, N-Methylpyrolidin, N,N,N',N'-Tetramethylethylendiamin, 1,5-Diazabicyclo(4,3,0)non-5-en oder 1,8-Diazabicyclo(5,4,0)undec-7-en.

Als Lösungsmittel kommen beispielsweise Halogenkohlenwasserstoffe wie z.B. Methylenchlorid, Chloroform, Tetrachlorkohlenstoff, 1,1,1-Trichlorethan oder Chlorbenzol;
Ether wie z.B. Diethylether, Methyl-tert.-butylether, Ethylenglycoldimethylether, Tetrahydrofuran oder Dioxan; Nitrokohlenwasserstoffe wie z.B. Nitrobenzol;

Nitrile wie z.B. Acetonitril oder Benzonitril; oder

Kohlenwasserstoffe wie z.B. Hexan, Heptan, Cyclohexan, Dekalin, Ligrin oder Toluol in Betracht.

Vorzugsweise arbeitet man ohne Lösungsmittel oder in Chlorbenzol oder Toluol ohne Base oder mit Triethylamin oder Pyridin, als Base.

Die Verbindungen Ib lassen sich beispielsweise aus den 4-Aryl-2-halogen-imidazol-5-carbonsäureestern Ia in an sich bekannter weise (Houben-Weyl, Bd. E5, S. 998ff) durch Alkylierung, Alkenylierung oder Alkinylierung erhalten.

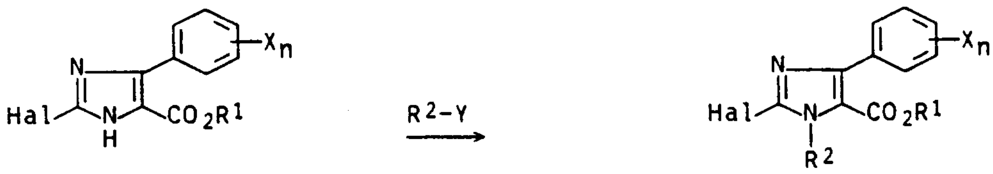


Ia VIII Ib

In der Formel VIII bedeutet Y beispielsweise eine leicht abspaltbare Gruppe wie Halogen, z.B. Chlorid, Bromid oder Iodid; Sulfonat wie z.B. Tosylat, Mesylat oder Triflat oder Alkylsulfat wie z.B. Methylsulfat oder Ethylsulfat.

Die Umsetzung von Ia mit VIII wird in einem Lösungsmittel in Gegenwart einer Base, drucklos oder unter Druck (1 bis 10 bar), kontinuierlich oder diskontinuierlich bei Temperaturen von 25 bis 200 °C, vorzugsweise -10 bis 150 °C durchgeführt.

Geeignete Basen hierbei sind z.B.

Alkaliverbindungen wie z.B. Natriumhydrid, Kaliumhydrid, Natriummethylat, Natriumethylat, Kalium-tert.butylat, Lithiumamid, Natrium- oder Kaliumhydroxid, Natriumcarbonat, Natriumbicarbonat oder Kaliumcarbonat oder

tertiäre Amine wie z.B. Triethylamin, N,N-Dimethyl-p-aminopyridin, Pyridin, N-Methylpyrolidin, Chinolin, N,N,N'-N'-Tetramethylethylendiamin, 1,5-Diazabicyclo(4,3,0)non-5-en oder 1,8-Diazabicyclo(5,4,0)undec-7-en.

Bevorzugt werden Natriumhydrid, Natriummethylat, Kalium-tert.-butylat oder 1,8-Diazabicyclo(5,4,0)-undec-7-en.

Geeignete Lösungensmittel sind beispielsweise Kohlenwasserstoffe wie Hexan, Heptan, Cyclohexan, Ligroin, Dekalin, Toluol oder Xylol; Ether wie z.B. Diethylether, Methyl-tert.-butylether, Ethylenglycoldimethylether, Tetrahydrofuran oder Dioxan; Amide wie z.B. Dimethylformamid; Sulfoxide wie z.B. Dimethylsulfoxid; Ketone wie z.B. Aceton oder Methylethylketon oder Alkohole wie z.B. Methanol, Ethanol oder i-Propanol.

Bevorzugt werden Tetrahydrofuran, Dimethylformamid, Toluol oder Dimethylsulfoxid.

In Hinblick auf die bestimmungsgemäße Verwendung der Verbindungen Ia und/oder Ib als pflanzenschützende Mittel kommen als Substituenten folgende Reste in Betracht:

$R^1$ Alkylgruppen wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 2,2-Dimethylpropyl, 1,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 2,2-Dimethylbutyl, 3,3-Dimethylbutyl, 1,2-Dimethylbutyl, 1,3-Dimethylbutyl 2,3-Dimethylbutyl, 1-Ethylbutyl oder 2-Ethylbutyl oder 1-Propylpropyl, besonders aber Methyl, Ethyl, Propyl oder 1-Methylethyl,

Alkenylgruppen wie z.B. 2-Propenyl, 2-Butenyl, 3-Butenyl, 1-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 2-Hexenyl, 3-Hexenyl, 4-Hexenyl, 5-Hexenyl oder 1-Methyl-2-hexenyl, besonders aber die 2-Propenylgruppe oder

Alkinylgruppen wie z.B. 2-Propinyl, 2-Butinyl, 3-Butinyl, 1-Methyl-2-propinyl, 2-Pentinyl, 3-Pentinyl, 4-Pentinyl oder 2-Hexinyl, besonders aber die 2-Propinylgruppe;

$R^2$ Alkylgruppen wie die unter $R^1$ genannten Reste, besonders aber Methyl, Ethyl oder 1-Methylethyl;

Alkenylgruppen wie die unter $R^1$ genannten Reste, besonders aber 2-Propenyl oder

Alkinylgruppen wie die unter $R^1$ genannten Reste, besonders die 2-Propinylgruppe;

X Cyano- oder Nitrogruppen,

Alkylgruppen wie Methyl, Ethyl, Propyl, 1-Methylethyl, Butyl, 1-Methylpropyl, 2-Methylpropyl oder 1,1-Dimethylethyl, besonders Methyl, 1-Methylpropyl und 1,1-Dimethylethyl;

Halogenalkylgruppen wie Trifluormethyl, Difluormethyl, Fluormethyl, Trichlormethyl, Dichlormethyl, Chlormethyl, 2,2,2-Trifluorethyl, 2,2-Difluorethyl, 2-Fluorethyl, 2,2,2-Trichlorethyl, 2-Chlorethyl oder 2-Bromethyl, besonders Trifluormethyl, Difluormethyl oder 2,2,2-Trifluormethyl;
Alkoxygruppen wie Methoxy, Ethoxy, Propoxy, 1-Methylethoxy, Butoxy, 1-Methylbutoxy, 2-Methylbutoxy oder 1,1-Dimethylethoxy, besonders Methoxy, Ethoxy oder 2-Propoxy;
Halogenalkoxygruppen wie Trifluormethoxy, Trichlormethoxy, Fluormethoxy, 2,2,2-Trifluorethoxy, 1,2,2-Trifluormethoxy oder 1,1,2,2-Tetrafluorethoxy, besonders Trifluormethoxy oder 2,2,2-Trifluorethoxy;
Alkylthiogruppen wie Methylthio, Ethylthio, Propylthio, 2-Propylthio, Butylthio, 1-Methylpropylthio, 2-Methylpropylthio oder 1,1-Dimethylethylthio, besonders Methylthio oder Ethylthio,
Alkylsulfinylgruppen wie Methylsulfinyl, Ethylsulfinyl,
Alkylsulfonylgruppen wie Methylsulfonyl, Ethylsulfonyl,
Halogenalkylthiogruppen wie Trifluormethylthio, Trichlormethylthio,
Carboalkoxygruppen wie Carbomethoxy, Carboethoxy,
N,N-Dialkylcarbamidogruppen wie N,N-Dimethylcarbamido, N,N-Diethylcarbamido oder
Halogenatome wie Fluor, Chlor, Brom oder Jod, besonders Fluor, Chlor oder Brom

n den Wert 0, 1, 2 oder 3, insbesondere 0, 1 oder 2.

Spezielle Beispiele für Cyclohexenone der Formel X, deren Kulturpflanzenverträglichkeit durch Halogenimidazolcarbonsäureester der Formeln Ia und Ib verbessert werden kann, sind in der folgenden Tabelle B aufgeführt.

Tabelle B

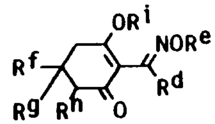 X

| Nr. | $R^d$ | $R^e$ | $R^f$ | $R^g$ | $R^h$ | $R^i$ | Literatur |
|---|---|---|---|---|---|---|---|
| X.1 | $C_3H_7$ | $CH_2CH=CH_2$ | $CH_3$ | $CH_3$ | $CO_2CH_3$ | Na | DE-A 2 439 104 |
| X.2 | $C_3H_7$ | $CH_2CH_3$ | $CH_2CH(CH_3)SCH_2CH_3$ | H | H | H | DE-A 2 822 304 |
| X.3 | $C_2H_5$ | $CH_2CH=CHCl$ | $CH_2CH(CH_3)SCH_2CH_3$ | H | H | H | US-A 4 440 566 |
| X.4 | $C_3H_7$ | $CH_2CH=CHCl$ | $CH_2CH(CH_3)SCH_2CH_3$ | H | H | H | US-A 4 440 566 |
| X.5 | $C_3H_7$ | $C_2H_5$ | 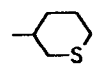 | H | H | H | EP-A 71 707 |
| X.6 | $C_2H_5$ | $C_2H_5$ | 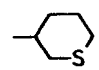 | H | H | H | EP-A 71 707 |

| Nr. | $R^d$ | $R^e$ | $R^f$ | $R^g$ | $R^h$ | $R^i$ | Literatur |
|---|---|---|---|---|---|---|---|
| X.7 | $CH_3$ | $CH_2CH=CHCH_3$ | 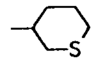 | H | H | H | EP-A 71 707 |
| X.8 | $C_3H_7$ | $C_2H_5$ | 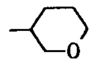 | H | H | H | EP-A 71 707 |
| X.9 | $C_2H_5$ | $CH_2CH=CHCl$ | 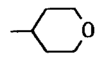 | H | H | H | EP-A 142 741 |
| X.10 | $C_3H_7$ | $C_2H_5$ | 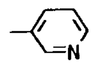 | H | H | H | EP-A 66 195 |
| X.11 | $C_2H_5$ | $C_2H_5$ | 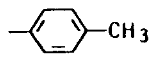 | H | H | H | DE-A 24 39 104 |
| X.12 | $C_2H_5$ | $CH_2CH=CHCH_3$ | 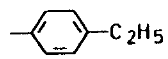 | H | H | H | Deutsche Anmeldung P 38 08 072.9 |
| X.13 | $C_2H_5$ | $C_2H_5$ | 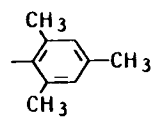 | H | H | H | EP-A 880 301 |
| X.14 | $C_3H_7$ | $CH_2CH=CHCl$ | 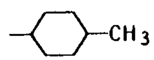 | H | H | H | EP-A 88 299 |
| X.15 | $C_3H_7$ | $CH_2CH=CHCH_3$ | 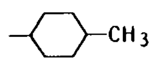 | H | H | H | EP-A 88 299 |

| Nr. | $R^d$ | $R^e$ | $R^f$ | $R^g$ | $R^h$ | $R^i$ | Literatur |
|---|---|---|---|---|---|---|---|
| X.16 | $C_2H_5$ | $CH_2CH{=}CHCH_3$ | 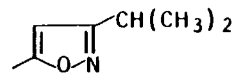 | H | H | H | EP-A 238 021 |
| X.17 | $C_3H_7$ | $CH_2CH{=}CHCH_3$ | 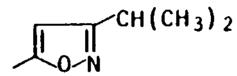 | H | H | H | EP-A 238 021 |
| X.18 | $C_2H_5$ | $CH_2CH{=}CHCl$ | 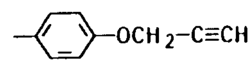 | H | H | H | EP-A 137 174 |
| X.19 | $C_3H_7$ | $C_2H_5$ | 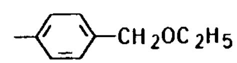 | H | H | H | EP-A 2 137 200 |
| X.20 | $C_3H_7$ | $C_2H_5$ | 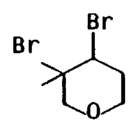 | H | H | H | EP-A 230 235 |
| X.21 | $C_3H_7$ | $CH_2CH{=}CHCl$ | 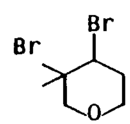 | H | H | H | EP-A 230 235 |

| Nr. | $R^d$ | $R^e$ | $R^f$ | $R^g$ | $R^h$ | $R^i$ | Literatur |
|---|---|---|---|---|---|---|---|
| X.22 | $C_3H_7$ | $CH_2CH=CHCl$ | 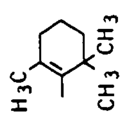 | H | H | H | EP-A 46 860 |
| X.23 | $C_3H_7$ | $C_2H_5$ | 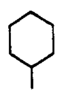 | H | H | H | JP-A 540 191 945 |
| X.24 | $C_3H_7$ | $C_2H_5$ | 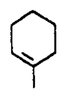 | H | H | H | EP-A 46 860 |
| X.25 | $CH_3$ | $CH_2CH=CHCl$ | 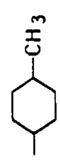 | H | H | H | EP-A 88 299 |
| X.26 | $C_3H_7$ | $C_2H_5$ | 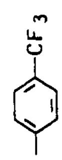 | H | H | K | EP-A 137 174 |
| X.27 | $C_2H_5$ | $CH_2CH=CHCl$ | 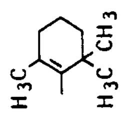 | H | H | H | EP-A 46 860 |

Herbizide Wirkstoffe und antidotisch wirkende Verbindungen können gemeinsam oder getrennt nach dem Auflaufen auf die Blätter und Sprosse der Kulturpflanzen und der unerwünschten Gräser ausgebracht werden. Bevorzugt wird das antidotisch wirkende Mittel gleichzeitig mit dem herbiziden Wirkstoff ausgebracht. Auch eine getrennt Ausbringung, wobei das Antidot zuerst und anschließend der herbizide Wirkstoff auf das Feld gebracht werden, ist möglich. Herbizider Wirkstoff und Antidot können hierbei als Spritzmittel

in suspendierbarer, emulgierbarer oder löslicher Form gemeinsam oder getrennt formuliert vorliegen.

Möglich ist auch eine Behandlung der Kulturpflanzensamen mit dem Antidot vor der Aussaat. Der herbizide Wirkstoff wird dann allein in der üblichen Weise appliziert.

Für das gleiche Cyclohexenonderivat X werden unterschiedliche Mengen einer antidotisch wirkenden Verbindung benötigt, wenn das Cyclohexenonderivat in verschiedenen Kulturen eingesetzt wird. Die Mengenverhältnisse, in denen ein Cyclohexenonderivat und ein Halogenimidazolcarbonsäureester der Formel Ia und/oder Ib eingesetzt werden, sind in breiten Bereichen variabel. Sie sind abhängig von der Struktur des Cyclohexenonderivats, des Halogenimidazolcarbonsäureesters der Formel Ia und/oder Ib und der jeweiligen Kultur. Geeignete Anteilverhältnisse herbizider Wirkstoff : antidotisch wirkender Verbindung liegen zwischen 1:4 bis 1:0,01; vorzugsweise 1:4 bis 1:0,25 Gew.-Teile.

Die neuen herbiziden Mittel können neben dem Halogenimidazolcarbonsäureester der Formel Ia und/oder Ib als Antidot und dem Herbizid aus der Gruppe der Cyclohexenone X weitere herbizide und wachstumsregulierende Wirkstoffe anderer chemischer Strukturen enthalten, wobei der antagonistische Effekt erhalten bleibt.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentigen wäßrigen, öligen oder sonstigen Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Ölsdispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure` Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalin derivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Synthesebeispiele:

Die in den nachstehenden Beispielen wiedergegebenen Vorschriften wurden unter entsprechender Abwandlung der Ausgangsverbindungen zur Gewinnung weiterer Verbindungen der Formeln Ia und Ib benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellten und untersuchten Verbindungen eine gleichartige Wirkung erwarten.

Beispiel 1

a) 4-Phenyl-2-imidazolon-5-carbonsäureethylester

Zu 112 g (0,582 mol) Benzoylessigsäureethylester in 130 ml Essigsäure wurden bei 35-45 °C eine Lösung von 35 g Natriumnitrit in 100 ml Wasser gegeben, eine 1/2 Stunde bei dieser Temperatur nachgerührt und anschließend mit 500 ml Wasser versetzt.

Nach 1 Stunde Rühren wurden 302 g Natriumdithionit in 600 ml Wasser zugesetzt, 1/2 Stunde nachgerührt und dann 157 g Kaliumcyanat zugegeben. Die Lösung wurde kurz auf 70-80 °C aufgeheizt und dann bei Raumtemperatur über Nacht stehengelassen. Extraktion mit Essigester lieferte 4-Phenyl-2-imidazolon-5-carbonsäureethylester vom Fp.: 109 - 112 °C.

b) 2-Chlor-4-phenylimidazol-5-carbonsäureethylester

20 g (0,086 mol) 4-Phenyl-2-imidazolon-5-carbonsäureethylester wurden mit 250 ml Phosphoroxychlorid 5 Stunden zum Rückfluß erhitzt. Das überschüssige Phosphoroxychlorid wurde im Vakuum abgezogen, der Rückstand auf Eis gegeben, mit Ammoniak auf pH 5 - 6 eingestellt, mit Essigester extrahiert, getrocknet und eingeengt. Chromatographie des Rückstandes an Kieselgel (Laufmittel Essigester/Pentan) lieferte 2-Chlor-4-phenylimidazol-5-carbonsäureethylester (Verbindung Nr. 1.001) vom Fp. 128 - 129 °C.

Beispiel 2

2-Chlor-1-methyl-4-phenylimidazol-5-carbonsäureethylester

Eine Lösung von 7 g (0,028 mol) 2-Chlor-4-phenylimidazol-5-carbonsäureethylester in 25 ml Dimethylformamid wurde mit 0,6 g Natriumhydrid versetzt. Nach Beendigung der Gasentwicklung wurden 4,2 g (0,033 mol) Dimethylsulfat zugegeben und die Mischung 1 1/2 Stunden auf 80 °C erhitzt. Das Dimethylformamid wurde im Vakuum abgezogen und der Rückstand auf verdünntes Ammoniakwasser gegeben. Nach Extraktion mit Essigester, Trocknung und Entfernung des Lösungsmittels ergab die Chromatographie an Kieselgel (Laufmittel Essigester/Pentan) 2-Chlor-1-methyl-4-phenylimidazol-5-carbonsäureethylester (Verbindung Nr. 2.001) vom Fp. 54 - 55 °C.

Tabelle 1

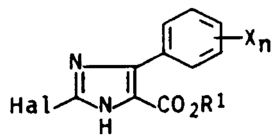

| Verb.-Nr. | Hal | $X_n$ | $R^1$ | Fp [°C] |
|---|---|---|---|---|
| 1.001 | Cl | H | $CH_2CH_3$ | 128-129 |
| 1.002 | Cl | H | $CH_3$ | |
| 1.003 | Cl | H | $CH_2CH_2CH_3$ | |
| 1.004 | Cl | H | $CH(CH_3)_2$ | |
| 1.005 | Cl | 2-F | $CH_3$ | |
| 1.006 | Cl | 2-F | $CH_2CH_3$ | 130-132 |
| 1.007 | Cl | 3-F | $CH_3$ | |
| 1.008 | Cl | 3-F | $CH_2CH_3$ | |
| 1.009 | Cl | 4-F | $CH_3$ | |
| 1.010 | Cl | 4-F | $CH_2CH_3$ | 77- 80 |
| 1.011 | Cl | 2-Cl | $CH_3$ | |
| 1.012 | Cl | 2-Cl | $CH_2CH_3$ | |
| 1.013 | Cl | 3-Cl | $CH_3$ | |
| 1.014 | Cl | 3-Cl | $CH_2CH_3$ | |
| 1.015 | Cl | 4-Cl | $CH_3$ | |
| 1.016 | Cl | 4-Cl | $CH_2CH_3$ | |
| 1.017 | Cl | 2-Br | $CH_3$ | |
| 1.018 | Cl | 2-Br | $CH_2CH_3$ | |
| 1.019 | Cl | 3-Br | $CH_3$ | |
| 1.020 | Cl | 3-Br | $CH_2CH_3$ | |
| 1.021 | Cl | 4-Br | $CH_3$ | |
| 1.022 | Cl | 4-Br | $CH_2CH_3$ | |
| 1.023 | Cl | 2-F, 4-F | $CH_3$ | |
| 1.024 | Cl | 2-F, 4-F | $CH_2CH_3$ | |
| 1.025 | Cl | 2-F, 4-F | $CH_2CH_2CH_3$ | |
| 1.026 | Cl | 2-F, 4-F | $CH_2CH{=}CH_2$ | |
| 1.027 | Cl | 2-F, 4-F | $CH_2C{\equiv}CH$ | |
| 1.028 | Cl | 3-F, 4-F | $CH_3$ | |
| 1.029 | Cl | 3-F, 4-F | $CH_2CH_3$ | |
| 1.030 | Cl | 2-F, 3-F | $CH_3$ | |
| 1.031 | Cl | 2-F, 3-F | $CH_2CH_3$ | |
| 1.032 | Cl | 2-F, 5-F | $CH_3$ | |
| 1.033 | Cl | 2-F, 5-F | $CH_2CH_3$ | |
| 1.034 | Cl | 2-F, 6-F | $CH_3$ | |
| 1.035 | Cl | 2-F, 6-F | $CH_2CH_3$ | |

Tabelle 1 (Fortsetzung)

| Verb.-Nr. | Hal | $X_n$ | $R^1$ | Fp [°C] |
|---|---|---|---|---|
| 1.036 | Cl | 2-Cl,3-Cl | $CH_3$ | |
| 1.037 | Cl | 2-Cl,3-Cl | $CH_2CH_3$ | |
| 1.038 | Cl | 2-Cl,4-Cl | $CH_3$ | |
| 1.039 | Cl | 2-Cl,4-Cl | $CH_2CH_3$ | |
| 1.040 | Cl | 2-Cl,4-Cl | $CH_2CH_2CH_3$ | |
| 1.041 | Cl | 2-Cl,4-Cl | $CH(CH_3)_2$ | |
| 1.042 | Cl | 2-Cl,4-Cl | $CH_2CH{=}CH_2$ | |
| 1.043 | Cl | 2-Cl,4-Cl | $CH_2C{\equiv}CH$ | |
| 1.044 | Cl | 2-Cl,5-Cl | $CH_3$ | |
| 1.045 | Cl | 2-Cl,5-Cl | $CH_2CH_3$ | |
| 1.046 | Cl | 2-Cl,6-Cl | $CH_3$ | |
| 1.047 | Cl | 2-Cl,6-Cl | $CH_2CH_3$ | |
| 1.048 | Cl | 3-Cl,4-Cl | $CH_3$ | |
| 1.049 | Cl | 3-Cl,4-Cl | $CH_2CH_3$ | |
| 1.050 | Cl | 3-Cl,5-Cl | $CH_3$ | |
| 1.051 | Cl | 3-Cl,5-Cl | $CH_2CH_3$ | |
| 1.052 | Cl | 2-Cl,4-Cl,6-Cl | $CH_3$ | |
| 1.053 | Cl | 2-Cl,4-Cl,6-Cl | $CH_2CH_3$ | |
| 1.054 | Cl | 2-Br,4-Br | $CH_3$ | |
| 1.055 | Cl | 2-Br,4-Br | $CH_2CH_3$ | |
| 1.056 | Cl | 2-F,4-Cl | $CH_3$ | |
| 1.057 | Cl | 2-F,4-Cl | $CH_2CH_3$ | |
| 1.058 | Cl | 2-$NO_2$ | $CH_3$ | |
| 1.059 | Cl | 2-$NO_2$ | $CH_2CH_3$ | |
| 1.060 | Cl | 3-$NO_2$ | $CH_3$ | |
| 1.061 | Cl | 3-$NO_2$ | $CH_2CH_3$ | |
| 1.062 | Cl | 4-$NO_2$ | $CH_3$ | |
| 1.063 | Cl | 4-$NO_2$ | $CH_2CH_3$ | |
| 1.064 | Cl | 2-$NO_2$,4-$NO_2$ | $CH_3$ | |
| 1.065 | Cl | 2-$NO_2$,4-$NO_2$ | $CH_2CH_3$ | |
| 1.066 | Cl | 2-Cl,4-$NO_2$ | $CH_3$ | |
| 1.067 | Cl | 2-Cl,4-$NO_2$ | $CH_2CH_3$ | |
| 1.068 | Cl | 2-$NO_2$,4-Cl | $CH_3$ | |
| 1.069 | Cl | 2-$NO_2$,4-Cl | $CH_2CH_3$ | |
| 1.070 | Cl | 2-CN | $CH_3$ | |
| 1.071 | Cl | 2-CN | $CH_2CH_3$ | |
| 1.072 | Cl | 4-CN | $CH_3$ | |
| 1.073 | Cl | 4-CN | $CH_2CH_3$ | 172 - 173 |
| 1.074 | Cl | 2-Cl,4-CN | $CH_3$ | |
| 1.075 | Cl | 2-Cl,4-CN | $CH_2CH_3$ | |

Tabelle 1 (Fortsetzung)

| Verb.-Nr. | Hal | $X_n$ | $R^1$ | Fp [°C] |
|---|---|---|---|---|
| 1.076 | Cl | 2-F, 4-CN | $CH_3$ | |
| 1.077 | Cl | 2-F, 4-CN | $CH_2CH_3$ | |
| 1.078 | Cl | 2-$CH_3$ | $CH_3$ | |
| 1.079 | Cl | 2-$CH_3$ | $CH_2CH_3$ | |
| 1.080 | Cl | 3-$CH_3$ | $CH_3$ | |
| 1.081 | Cl | 3-$CH_3$ | $CH_2CH_3$ | |
| 1.082 | Cl | 4-$CH_3$ | $CH_3$ | |
| 1.083 | Cl | 4-$CH_3$ | $CH_2CH_3$ | 150-151 |
| 1.084 | Cl | 4-$C(CH_3)_3$ | $CH_3$ | |
| 1.085 | Cl | 4-$C(CH_3)_3$ | $CH_2CH_3$ | |
| 1.086 | Cl | 2-$CH_3$, 4-$CH_3$ | $CH_3$ | |
| 1.087 | Cl | 2-$CH_3$, 4-$CH_3$ | $CH_2CH_3$ | |
| 1.088 | Cl | 2-$CH_3$, 6-$CH_3$ | $CH_3$ | |
| 1.089 | Cl | 2-$CH_3$, 6-$CH_3$ | $CH_2CH_3$ | |
| 1.090 | Cl | 2-$CH_3$, 4-$CH_3$, 6-$CH_3$ | $CH_3$ | |
| 1.091 | Cl | 2-$CH_3$-4-$CH_3$, 6-$CH_3$ | $CH_2CH_3$ | |
| 1.092 | Cl | 2-$CF_3$ | $CH_3$ | |
| 1.093 | Cl | 2-$CF_3$ | $CH_2CH_3$ | |
| 1.094 | Cl | 3-$CF_3$ | $CH_3$ | |
| 1.095 | Cl | 3-$CF_3$ | $CH_2CH_3$ | |
| 1.096 | Cl | 4-$CF_3$ | $CH_3$ | |
| 1.097 | Cl | 4-$CF_3$ | $CH_2CH_3$ | |
| 1.098 | Cl | 2-Cl, 4-$CF_3$ | $CH_3$ | |
| 1.099 | Cl | 2-Cl, 4-$CF_3$ | $CH_2CH_3$ | |
| 1.100 | Cl | 2-F, 4-$CF_3$ | $CH_3$ | |
| 1.101 | Cl | 2-F, 4-$CF_3$ | $CH_2CH_3$ | |
| 1.102 | Cl | 4-$CF_2Cl$ | $CH_3$ | |
| 1.103 | Cl | 4-$CF_2Cl$ | $CH_2CH_3$ | |
| 1.104 | Cl | 4-$OCF_2CHF_2$ | $CH_3$ | |
| 1.105 | Cl | 4-$OCF_2CHCF_2$ | $CH_2CH_3$ | |
| 1.106 | Cl | 4-$OCF_3$ | $CH_3$ | |
| 1.107 | Cl | 4-$OCF_3$ | $CH_2CH_3$ | |
| 1.108 | Cl | 2-$OCH_3$ | $CH_3$ | |
| 1.109 | Cl | 2-$OCH_3$ | $CH_2CH_3$ | |
| 1.110 | Cl | 3-$OCH_3$ | $CH_3$ | |
| 1.111 | Cl | 3-$OCH_3$ | $CH_2CH_3$ | |
| 1.112 | Cl | 4-$OCH_3$ | $CH_3$ | |
| 1.113 | Cl | 4-$OCH_3$ | $CH_2CH_3$ | |
| 1.114 | Cl | 2-$OCH_3$, 4-$OCH_3$ | $CH_3$ | |
| 1.115 | Cl | 2-$OCH_3$, 4-$OCH_3$ | $CH_2CH_3$ | |

Tabelle 1 (Fortsetzung)

| Verb.-Nr. | Hal | $X_n$ | $R^1$ | Fp [°C] |
|---|---|---|---|---|
| 1.116 | Cl | 2-$OCH_3$,6-$OCH_3$ | $CH_3$ | |
| 1.117 | Cl | 2-$OCH_3$,6-$OCH_3$ | $CH_2CH_3$ | |
| 1.118 | Cl | 4-$OCH_2CH_3$ | $CH_3$ | |
| 1.119 | Cl | 4-$OCH_2CH_3$ | $CH_2CH_3$ | |
| 1.120 | Cl | 2-$SCH_3$ | $CH_3$ | |
| 1.121 | Cl | 2-$SCH_3$ | $CH_2CH_3$ | |
| 1.122 | Cl | 4-$SCH_3$ | $CH_3$ | |
| 1.123 | Cl | 4-$SCH_3$ | $CH_2CH_3$ | |
| 1.124 | Cl | 2-$SOCH_3$ | $CH_3$ | |
| 1.125 | Cl | 2-$SOCH_3$ | $CH_2CH_3$ | |
| 1.126 | Cl | 2-$SO_2CH_3$ | $CH_3$ | |
| 1.127 | Cl | 2-$SO_2CH_3$ | $CH_2CH_3$ | |
| 1.128 | Cl | 2-$CO_2CH_3$ | $CH_3$ | |
| 1.129 | Cl | 2-$CO_2CH_3$ | $CH_2CH_3$ | |
| 1.130 | Cl | 2-$CON(CH_3)_2$ | $CH_3$ | |
| 1.131 | Cl | 2-$CON(CH_3)_2$ | $CH_2CH_3$ | |
| 1.132 | Br | H | $CH_3$ | |
| 1.133 | Br | H | $CH_2CH_3$ | |
| 1.134 | Br | 4-F | $CH_2CH_3$ | |
| 1.135 | Br | 4-Cl | $CH_3$ | |
| 1.136 | Br | 4-Cl | $CH_2CH_3$ | |
| 1.137 | Br | 4-Br | $CH_2CH_3$ | |
| 1.138 | Br | 2-Cl,4-Cl | $CH_3$ | |
| 1.139 | Br | 2-Cl,4-Cl | $CH_2CH_3$ | |
| 1.140 | Br | 2-Cl,6-Cl | $CH_3$ | |
| 1.141 | Br | 2-Cl,6-Cl | $CH_2CH_3$ | |
| 1.142 | Br | 2-F,4-F | $CH_3$ | |
| 1.143 | Br | 2-F,4-F | $CH_2CH_3$ | |
| 1.144 | Br | 2-F,6-F | $CH_2CH_3$ | |
| 1.145 | Br | 2-Cl,4-Cl,6-Cl | $CH_2CH_3$ | |
| 1.146 | Br | 4-$NO_2$ | $CH_2CH_3$ | |
| 1.147 | Br | 2-$NO_2$,4-$NO_2$ | $CH_2CH_3$ | |
| 1.148 | Br | 2-Cl,4-$NO_2$ | $CH_2CH_3$ | |
| 1.149 | Br | 2-$NO_2$,4-Cl | $CH_2CH_3$ | |
| 1.150 | Br | 2-$CH_3$,4-$CH_3$ | $CH_2CH_3$ | |
| 1.151 | Br | 2-$CH_3$,6-$CH_3$ | $CH_2CH_3$ | |
| 1.152 | Br | 4-$CF_3$ | $CH_2CH_3$ | |
| 1.153 | Br | 3-$CF_3$ | $CH_2CH_3$ | |
| 1.154 | Br | 2-F,4-$CF_3$ | $CH_2CH_3$ | |

Tabelle 1 (Fortsetzung)

| Verb.-Nr. | Hal | $X_n$ | $R^1$ | Fp [$^oC$] |
|---|---|---|---|---|
| 1.155 | Br | 2-$OCH_3$, 4-$OCH_3$ | $CH_2CH_3$ | |
| 1.156 | Br | 2-$OCH_3$, 6-$OCH_3$ | $CH_2CH_3$ | |
| 1.157 | Br | 4-$SCF_3$ | $CH_2CH_3$ | |

Tabelle 2

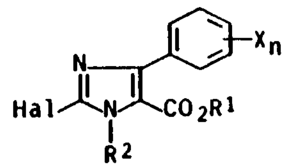

| Verb.-Nr. | Hal | $X_n$ | $R^1$ | $R^2$ | Fp [°C] |
|---|---|---|---|---|---|
| 2.001 | Cl | H | $CH_2CH_3$ | $CH_3$ | 54- 55 |
| 2.002 | Cl | H | $CH_2CH_3$ | $CH_2CH_3$ | |
| 2.003 | Cl | H | $CH_2CH_3$ | $CH_2CH_2CH_3$ | |
| 2.004 | Cl | H | $CH_2CH_3$ | $CH(CH_3)_2$ | |
| 2.005 | Cl | H | $CH_3$ | $CH_3$ | |
| 2.006 | Cl | H | $CH_3$ | $CH(CH_3)_2$ | |
| 2.007 | Cl | 2-F | $CH_2CH_3$ | $CH_3$ | |
| 2.008 | Cl | 2-F | $CH_2CH_3$ | $CH_2CH=CH_2$ | |
| 2.009 | Cl | 3-F | $CH_2CH_3$ | $CH_3$ | |
| 2.010 | Cl | 4-F | $CH_2CH_3$ | $CH_3$ | Öl [$^1H$-NMR($d_6$-DMSO): 3,83(s,3H); 4,08-4,15(q,2H)] |
| 2.011 | Cl | 4-F | $CH_2CH_3$ | $CH_2CH_3$ | |
| 2.012 | Cl | 4-F | $CH_3$ | $CH_3$ | |
| 2.013 | Cl | 2-Cl | $CH_3$ | $CH_3$ | |
| 2.014 | Cl | 2-Cl | $CH_2CH_3$ | $CH_3$ | |
| 2.015 | Cl | 4-Cl | $CH_3$ | $CH_3$ | |
| 2.016 | Cl | 4-Cl | $CH_2CH_3$ | $CH_3$ | |
| 2.017 | Cl | 4-Br | $CH_3$ | $CH_3$ | |

Tabelle 2 (Fortsetzung)

| Verb.-Nr. | Hal | $X_n$ | $R^1$ | $R^2$ | Fp [°C] |
|---|---|---|---|---|---|
| 2.018 | Cl | 4-Br | $CH_2CH_3$ | $CH_3$ | |
| 2.019 | Cl | 2-F,4-F | $CH_3$ | $CH_3$ | |
| 2.020 | Cl | 2-F,4-F | $CH_3$ | $CH_2C{\equiv}CH$ | |
| 2.021 | Cl | 2-F,4-F | $CH_3$ | $CH(CH_3)_2$ | |
| 2.022 | Cl | 2-F,4-F | $CH_2CH_3$ | $CH_3$ | |
| 2.023 | Cl | 2-F,4-F | $CH_2CH_3$ | $CH_2CH_3$ | |
| 2.024 | Cl | 2-F,4-F | $CH_2CH_3$ | $CH(CH_3)_2$ | |
| 2.025 | Cl | 3-F,4-F | $CH_2CH_3$ | $CH_3$ | |
| 2.026 | Cl | 2-F,3-F | $CH_2CH_3$ | $CH_3$ | |
| 2.027 | Cl | 2-F,5-F | $CH_2CH_3$ | $CH_3$ | |
| 2.028 | Cl | 2-F,6-F | $CH_3$ | $CH_3$ | |
| 2.029 | Cl | 2-F,6-F | $CH_2CH_3$ | $CH_3$ | |
| 2.030 | Cl | 2-F,6-F | $CH_2CH_3$ | $CH_2CH_3$ | |
| 2.031 | Cl | 2-F,6-F | $CH_2CH_3$ | $CH(CH_3)_2$ | |
| 2.032 | Cl | 2-Cl,3-Cl | $CH_3$ | $CH_3$ | |
| 2.033 | Cl | 2-Cl,3-Cl | $CH_2CH_3$ | $CH_3$ | |
| 2.034 | Cl | 2-Cl,4-Cl | $CH_3$ | $CH_3$ | |
| 2.035 | Cl | 2-Cl,4-Cl | $CH_3$ | $CH_2CH_3$ | |
| 2.036 | Cl | 2-Cl,4-Cl | $CH_3$ | $CH(CH_3)_2$ | |
| 2.037 | Cl | 2-Cl,4-Cl | $CH_2CH_3$ | $CH_3$ | |
| 2.038 | Cl | 2-Cl,4-Cl | $CH_2CH_3$ | $CH_2CH_3$ | |
| 2.039 | Cl | 2-Cl,4-Cl | $CH_2CH_3$ | $CH(CH_3)_2$ | |
| 2.040 | Cl | 2-Cl,4-Cl | $CH_2CH=CH_2$ | $CH_3$ | |
| 2.041 | Cl | 2-Cl,4-Cl | $CH_2CH=CH_2$ | $CH_2CH_3$ | |

Tabelle 2 (Fortsetzung)

| Verb.-Nr. | Hal | $X_n$ | $R^1$ | $R^2$ | Fp [°C] |
|---|---|---|---|---|---|
| 2.042 | Cl | 2-Cl, 4-Cl | $CH_2CH{=}CH_2$ | $CH(CH_3)_2$ | |
| 2.043 | Cl | 2-Cl, 4-Cl | $CH_2C{\equiv}CH$ | $CH_3$ | |
| 2.044 | Cl | 2-Cl, 4-Cl | $CH_2C{\equiv}CH$ | $CH_2CH_3$ | |
| 2.045 | Cl | 2-Cl, 4-Cl | $CH_2C{\equiv}CH$ | $CH(CH_3)_2$ | |
| 2.046 | Cl | 2-Cl, 5-Cl | $CH_3$ | $CH_3$ | |
| 2.047 | Cl | 2-Cl, 5-Cl | $CH_2CH_3$ | $CH_3$ | |
| 2.048 | Cl | 2-Cl, 6-Cl | $CH_3$ | $CH_3$ | |
| 2.049 | Cl | 2-Cl, 6-Cl | $CH_2CH_3$ | $CH_3$ | |
| 2.050 | Cl | 3-Cl, 4-Cl | $CH_3$ | $CH_3$ | |
| 2.051 | Cl | 3-Cl, 4-Cl | $CH_2CH_3$ | $CH_3$ | |
| 2.052 | Cl | 3-Cl, 5-Cl | $CH_2CH_3$ | $CH_3$ | |
| 2.053 | Cl | 2-Cl, 4-Cl, 6-Cl | $CH_3$ | $CH_3$ | |
| 2.054 | Cl | 2-Cl, 4-Cl, 6-Cl | $CH_2CH_3$ | $CH_3$ | |
| 2.055 | Cl | 2-Br, 4-Br | $CH_3$ | $CH_3$ | |
| 2.056 | Cl | 2-Br, 4-Br | $CH_3$ | $CH(CH_3)_2$ | |
| 2.057 | Cl | 2-Br, 4-Br | $CH_2CH_3$ | $CH_3$ | |
| 2.058 | Cl | 2-Br, 4-Br | $CH_2CH_3$ | $CH(CH_3)_2$ | |
| 2.059 | Cl | 2-F, 4-Cl | $CH_3$ | $CH_3$ | |
| 2.060 | Cl | 2-F, 4-Cl | $CH_2CH_3$ | $CH_3$ | |
| 2.061 | Cl | 2-$NO_2$ | $CH_2CH_3$ | $CH_3$ | |
| 2.062 | Cl | 3-$NO_2$ | $CH_2CH_3$ | $CH_3$ | |
| 2.063 | Cl | 4-$NO_2$ | $CH_3$ | $CH_3$ | |
| 2.064 | Cl | 4-$NO_2$ | $CH_2CH_3$ | $CH_3$ | |
| 2.065 | Cl | 2-$NO_2$, 4-$NO_4$ | $CH_3$ | $CH_3$ | |

Tabelle 2 (Fortsetzung)

| Verb.-Nr. | Hal | $X_n$ | $R^1$ | $R^2$ | Fp [°C] |
|---|---|---|---|---|---|
| 2.066 | Cl | $2-NO_2, 4-NO_4$ | $CH_3$ | $CH_2CH_3$ | |
| 2.067 | Cl | $2-NO_2, 4-NO_4$ | $CH_2CH_3$ | $CH_3$ | |
| 2.068 | Cl | $2-NO_2, 4-NO_4$ | $CH_2CH_3$ | $CH_2CH_3$ | |
| 2.069 | Cl | $2-Cl, 4-NO_2$ | $CH_3$ | $CH_3$ | |
| 2.070 | Cl | $2-Cl, 4-NO_2$ | $CH_2CH_3$ | $CH_3$ | |
| 2.071 | Cl | $2-NO_2, 4-Cl$ | $CH_3$ | $CH_3$ | |
| 2.072 | Cl | $2-NO_2, 4-Cl$ | $CH_2CH_3$ | $CH_3$ | |
| 2.073 | Cl | 2-CN | $CH_2CH_3$ | $CH_3$ | |
| 2.074 | Cl | 4-CN | $CH_3$ | $CH_3$ | |
| 2.075 | Cl | 4-CN | $CH_2CH_3$ | $CH_3$ | |
| 2.076 | Cl | 2-Cl, 4-CN | $CH_3$ | $CH_3$ | |
| 2.077 | Cl | 2-Cl, 4-CN | $CH_2CH_3$ | $CH_3$ | |
| 2.078 | Cl | 2-F, 4-CN | $CH_2CH_3$ | $CH_3$ | |
| 2.079 | Cl | $2-CH_3$ | $CH_2CH_3$ | $CH_3$ | |
| 2.080 | Cl | $3-CH_3$ | $CH_2CH_3$ | $CH_3$ | |
| 2.081 | Cl | $4-CH_3$ | $CH_3$ | $CH_3$ | |
| 2.082 | Cl | $4-CH_3$ | $CH_2CH_3$ | $CH_3$ | 73- 75 |
| 2.083 | Cl | $2-CH_3, 4-CH_3$ | $CH_3$ | $CH_3$ | |
| 2.084 | Cl | $2-CH_3, 4-CH_3$ | $CH_2CH_3$ | $CH_3$ | |
| 2.085 | Cl | $2-CH_3, 6-CH_3$ | $CH_3$ | $CH_3$ | |
| 2.086 | Cl | $2-CH_3, 6-CH_3$ | $CH_2CH_3$ | $CH_3$ | |
| 2.087 | Cl | $2-CH_3, 4-CH_3, 6-CH_3$ | $CH_3$ | $CH_3$ | |
| 2.088 | Cl | $2-CH_3, 4-CH_3, 6-CH_3$ | $CH_2CH_3$ | $CH_3$ | |
| 2.089 | Cl | $2-CF_3$ | $CH_2CH_3$ | $CH_3$ | |

Tabelle 2 (Fortsetzung)

| Verb.-Nr. | Hal | $X_n$ | $R^1$ | $R^2$ | Fp [°C] |
|---|---|---|---|---|---|
| 2.090 | Cl | $3-CF_3$ | $CH_3$ | $CH_3$ | |
| 2.091 | Cl | $3-CF_3$ | $CH_2CH_3$ | $CH_3$ | |
| 2.092 | Cl | $4-CF_3$ | $CH_3$ | $CH_3$ | |
| 2.093 | Cl | $4-CF_3$ | $CH_2CH_3$ | $CH_3$ | |
| 2.094 | Cl | $2-Cl,4-CF_3$ | $CH_3$ | $CH_3$ | |
| 2.095 | Cl | $2-Cl,4-CF_3$ | $CH_3$ | $CH(CH_3)_2$ | |
| 2.096 | Cl | $2-Cl,4-CF_3$ | $CH_2CH_3$ | $CH_3$ | |
| 2.097 | Cl | $2-Cl,4-CF_3$ | $CH_2CH_3$ | $CH_2CH_3$ | |
| 2.098 | Cl | $4-OCF_3$ | $CH_2CH_3$ | $CH_3$ | |
| 2.099 | Cl | $2-OCH_3$ | $CH_2CH_3$ | $CH_3$ | |
| 2.100 | Cl | $3-OCH_3$ | $CH_2CH_3$ | $CH_3$ | |
| 2.101 | Cl | $4-OCH_3$ | $CH_3$ | $CH_3$ | |
| 2.102 | Cl | $4-OCH_3$ | $CH_2CH_3$ | $CH_3$ | |
| 2.103 | Cl | $2-OCH_3,4-OCH_3$ | $CH_3$ | $CH_3$ | |
| 2.104 | Cl | $2-OCH_3,4-OCH_3$ | $CH_2CH_3$ | $CH_3$ | |
| 2.105 | Cl | $2-OCH_3,6-OCH_3$ | $CH_3$ | $CH_3$ | |
| 2.106 | Cl | $2-OCH_3,6-OCH_3$ | $CH_2CH_3$ | $CH_3$ | |
| 2.107 | Cl | $2-SCH_3$ | $CH_2CH_3$ | $CH_3$ | |
| 2.108 | Cl | $4-SCH_3$ | $CH_2CH_3$ | $CH_3$ | |
| 2.109 | Cl | $2-SOCH_3$ | $CH_2CH_3$ | $CH_3$ | |
| 2.110 | Cl | $2-SO_2CH_3$ | $CH_2CH_3$ | $CH_3$ | |
| 2.111 | Cl | $2-CO_2CH_3$ | $CH_2CH_3$ | $CH_3$ | |
| 2.112 | Cl | $2-CON(CH_3)_2$ | $CH_2CH_3$ | $CH_3$ | |
| 2.113 | Br | H | $CH_2CH_3$ | $CH_3$ | |

Tabelle 2 (Fortsetzung)

| Verb.-Nr. | Hal | $X_n$ | $R^1$ | $R^2$ | Fp [°C] |
|---|---|---|---|---|---|
| 2.114 | Br | 4-F | $CH_2CH_3$ | $CH_3$ | |
| 2.115 | Br | 4-Cl | $CH_2CH_3$ | $CH_3$ | |
| 2.116 | Br | 4-Br | $CH_2CH_3$ | $CH_3$ | |
| 2.117 | Br | 2-Cl, 4-Cl | $CH_3$ | $CH_3$ | |
| 2.118 | Br | 2-Cl, 4-Cl | $CH_2CH_3$ | $CH_3$ | |
| 2.119 | Br | 2-Cl, 6-Cl | $CH_2CH_3$ | $CH_3$ | |
| 2.120 | Br | 2-F, 4-F | $CH_2CH_3$ | $CH_3$ | |
| 2.121 | Br | 2-F, 6-F | $CH_2CH_3$ | $CH_3$ | |
| 2.122 | Br | 2-Cl, 4-Cl, 6-Cl | $CH_2CH_3$ | $CH_3$ | |
| 2.123 | Br | 4-$NO_2$ | $CH_2CH_3$ | $CH_3$ | |
| 2.124 | Br | 2-$NO_2$, 4-$NO_2$ | $CH_2CH_3$ | $CH_3$ | |
| 2.125 | Br | 2-Cl, 4-$NO_2$ | $CH_2CH_3$ | $CH_3$ | |
| 2.126 | Br | 2-$NO_2$, 4-Cl | $CH_2CH_3$ | $CH_3$ | |
| 2.127 | Br | 2-$CH_3$, 4-$CH_3$ | $CH_2CH_3$ | $CH_3$ | |
| 2.128 | Br | 2-$CH_3$, 6-$CH_3$ | $CH_2CH_3$ | $CH_3$ | |
| 2.129 | Br | 3-$CF_3$ | $CH_2CH_3$ | $CH_3$ | |
| 2.130 | Br | 4-$CF_3$ | $CH_2CH_3$ | $CH_3$ | |
| 2.131 | Br | 2-F, 4-$CF_3$ | $CH_2CH_3$ | $CH_3$ | |
| 2.132 | Br | 2-$OCH_3$, 4-$OCH_3$ | $CH_2CH_3$ | $CH_3$ | |
| 2.133 | Br | 2-$OCH_3$-6-$OCH_3$ | $CH_2CH_3$ | $CH_3$ | |

Anwendungsbeispiele:

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und unerwünschten Pflanzen im Vergleich zum herbiziden Wirkstoff allein wird durch die folgenden biologischen Beispiele aus Gewächshausversuchen belegt:

Zur Anzucht der Testpflanzen dienen Plastikblumentöpfe mit rund 300 $cm^3$ Inhalt und lehmiger Sand mit etwa 3,0 % Humus als Substrat. Die Samen der Testpflanzen werden nach Arten getrennt, flach eingesät und befeuchtet. Danach deckt man die Gefäße mit durchsichtigen Plastikhauben ab, bis die Samen gleichmäßig gekeimt und die Pflanzen angewachsen sind.

Für die Nachauflaufbehandlung zieht man die Testpflanzen je nach Wuchsform erst bis zu einer Wuchshöhe von 3 bis 20 cm an und behandelt sie dann. Die herbiziden Mittel werden hierbei in Wasser als Verteilungsmittel suspendiert und emulgiert und mittels fein verteilender Düsen gespritzt.

Als herbizider Wirkstoff wurde in den biologischen Beispielen das Cyclohexenonderivat X.2 verwendet:

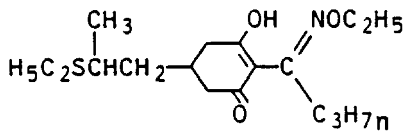

X.2

Die Formulierungen enthalten zwischen 0,1 und 95 Gew.-% an herbizidem Wirkstoff und Antidot, vorzugsweise zwischen 0,5 und 90 Gew.-%. Die Aufwandmengen an herbizidem Wirkstoff betragen 0,001 bis 0,5 kg/ha.

Der herbizide Wirkstoff X.2 wird als kommerziell formuliertes Produkt (184 g/l EC) auch allein jeweils unter Zugabe von derjenigen Menge an Lösungsmittelsystem XXII (Leerformulierung) in die Spritzbrühe eingesetzt, mit welcher die antidotisch wirkende Verbindung in den in den Tabellen angegebenen Aufwandmengen ausgebracht werden.

Sämtliche antidotisch wirkende Verbindungen werden in einem Gemisch, bestehend aus 80 % Cyclohexenonen und 20 % Emulphor EL (Versuchsformulierung XXII), mit 10 % Gew.-% Wirkstoff aufbereitet.

Die Versuchsgefäße werden im Gewächshaus aufgestellt, wobei für wärmeliebende Arten 18 bis 35° und für solche gemäßigtere Klimate 10 bis 25° bevorzugt werden.

Die Versuchsperiode erstreckte sich über 3 bis 5 Wochen. Während dieser Zeit wurden die Pflanzen gepflegt, und ihre Reaktion auf die einzelnen Behandlungen wurde erfaßt. Bewertet wurde die Schädigung durch die chemischen Mittel anhand einer Skala von 0 % bis 100 % im Vergleich zu den unbehandelten Kontrollpflanzen. Dabei bedeutet 0 keine Schädigung und 100 eine völlige Zerstörung der Pflanzen (Triticum aestivum, Weizen).

Die anschließende Tabelle dokumentiert die antidotische Wirkung, wobei die Beispielsverbindungen Nr. 1.001 die Kulturpflanzenverträglichkeit des Cyclohexenonderivats X.2 wesentlich verbessert.

Tabelle A

Verringerung der durch das Herbizid X.2 verursachten Schäden an Weizen durch Kombination mit der erfindungsgemäßen Verbindung 1.001.

| Aufwandmenge [kg/ha] | | Schädigung in % bei Weizen |
|---|---|---|
| Herbizider Wirkstoff X.2 | Antidotische Verbindung 1.001 | |
| 0.015 | - | 44 |
| 0.03 | - | 80 |
| 0.015 | 0.06 | 0 |
| 0.03 | 0.125 | 10 |

**Patentansprüche**

**1.** 4-Aryl-2-halogenimidazol-5-carbonsäureester der allgemeinen Formeln Ia und Ib

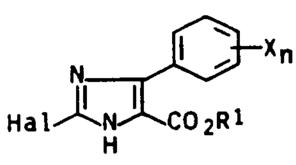

Ia

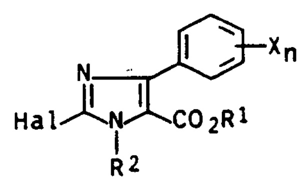

Ib

worin die Substituenten und Indizes die folgenden Bedeutungen haben:

$R^1$ eine $C_1$-$C_6$-Alkylgruppe, eine $C_3$-$C_6$-Alkenylgruppe oder eine $C_3$-$C_6$-Alkinylgruppe

- $R^2$ eine $C_1$-$C_6$-Alkylgruppe, eine $C_3$-$C_6$-Alkenylgruppe oder eine $C_3$-$C_6$-Alkinylgruppe,
- Hal Chlor oder Brom
- n 0 bis 3
- X eine $C_1$-$C_4$-Alkylgruppe, eine $C_1$-$C_4$-Halogenalkylgruppe, eine $C_1$-$C_4$-Alkoxygruppe, eine $C_1$-$C_4$-Halogenalkoxygruppe, eine $C_1$-$C_4$-Alkylthiogruppe, eine $C_1$-$C_4$-Alkylsulfinylgruppe, eine $C_1$-$C_4$-Alkylsulfonylgruppe, eine $C_1$-$C_4$-Halogenalkylthiogruppe, eine Cyanogruppe, eine Nitrogruppe, eine Carbo-$C_1$-$C_4$-alkoxygruppe, eine N,N-Di-$C_1$-$C_4$-alkylcarbamidogruppe und/oder ein Halogenatom.

**2.** Verfahren zur Herstellung der Verbindungen Ia gemäß Anspruch 1, dadurch gekennzeichnet, daß man eine Carbonylverbindung II

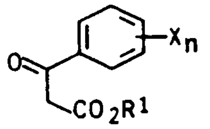

II

in an sich bekannter Weise ohne Isolierung der Zwischenstufen nacheinander in einer wäßrigen Säure zuerst mit einem Nitrit III,

$A^{\oplus}NO_2^{\ominus}$ III

worin $A^{\oplus}$ ein Alkalimetallion bedeutet, zu IV nitrosiert,

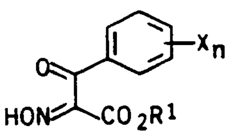

IV

danach zu V reduziert,

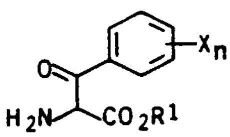

V

und direkt mit einem Cyanat VI,

$B^{\oplus}OCN^{\ominus}$ VI

worin $B^{\oplus}$ für ein Alkalimetallkation oder für Ammoniumkation steht, in Anwesenheit oder ohne Zusatz eines inerten organischen Lösungsmittels in ein Imidazolon VII

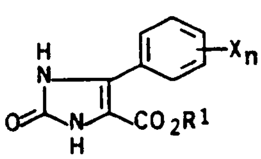

VII

überführt, und dieses anschließend mit einem Halogenierungsmittel zu Ia umsetzt.

**3.** Verfahren zur Herstellung der Verbindungen Ib, gemäß Anspruch 1, dadurch gekennzeichnet, daß man einen 4-Aryl-2-halogenimidazol-5-carbonsäureester Ia mit einem Alkylierungsmittel VIII,

$R^2$-Y VIII

worin Y Halogen, eine Sulfonat- oder eine Sulfatgruppe bedeutet, zu Ib umsetzt.

**4.** Herbizide Mittel, enthaltend mindestens einen 4-Aryl-2-halogenimidazol-5-carbonsäureester Ia und/oder Ib gemäß Anspruch 1 sowie mindestens einen herbiziden Wirkstoff aus der Gruppe der Cyclohexenonderivate der Formel X,

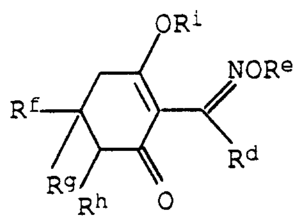

X

in welcher die Substituenten folgende Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkenylgruppe oder eine Thenylgruppe (=Thienylmethylgruppe), durch ein Halogenatom substituiert sein kann;

$R^f$ eine $C_1$-$C_4$-Alkylgruppe, welche einfach durch $C_1$-$C_4$-Alkylthio oder $C_1$-$C_4$-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;
einen 10-gliedrigen gesättigten oder einfach ungesättigten Heterocyclus, welcher zwei Sauerstoffatome oder Schwefelatome enthält und durch bis zu drei $C_1$-$C_4$-Alkylgruppen und/oder Methoxygruppen substituiert sein kann;
eine Phenylgruppe, eine Pyridylgruppe oder eine Isoxazolylgruppe, wobei diese Gruppen bis zu drei der folgenden Reste tragen können:
$C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino;

$R^g$ Wasserstoff, Hydroxy oder, wenn $R^f$ die Bedeutung $C_1$-$C_6$-Alkylgruppe hat, eine $C_1$-$C_6$-Alkylgruppe;

$R^h$ Wasserstoff, eine Cyanogruppe, ein Halogenatom oder eine $C_1$-$C_4$-Alkoxycarbonylgruppe und

$R^i$ Wasserstoff oder das Äquivalent eines umweltverträglichen Kations.

**5.** Herbizide Mittel nach Anspruch 4, enthaltend ein Halogenimidazolderivat Ia oder Ib sowie ein Herbizid der Formel X, wobei das Anteilsverhältnis Ia oder Ib zu X 4:1 bis 0,01:1 Gewichtsteile beträgt.

**6.** Verfahren zur selektiven Bekämpfung von unerwünschtem Pflanzenwuchs, dadurch gekennzeichnet, daß man ein Halogenimidazolderivat der Formel Ia und/oder Ib gemäß Anspruch 1 und ein Cyclohexenonderivat der Formel X gemäß Anspruch 4 vor, bei oder nach der Aussaat der Kulturpflanzen, vor oder während des Auflaufens der Kulturpflanzen gleichzeitig oder nacheinander ausbringt.

**7.** Verfahren zur Verhinderung von Schädigungen von Kulturpflanzen durch herbizide Cyclohexenonderivate der Formel X gemäß Anspruch 4, dadurch gekennzeichnet, daß man das Saatgut der Kulturpflanzen mit einer antagonistisch wirksamen Menge eines Halogenimidazolderivats der Formel Ia und/oder Ib gemäß Anspruch 1 behandelt.

**8.** Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit

einem Halogenimidazolderivat der Formel Ia und/oder Ib gemäß Anspruch 1 und mit Cyclohexenonderivat der Formel X gemäß Anspruch 4 gleichzeitig oder nacheinander behandelt.

9. Verfahren gemäß den Ansprüchen 6 bis 8, dadurch gekennzeichnet, daß die Kulturpflanzen Gerste, Weizen, Mais, Kultusorghum und Reis sind.

## Claims

1. A 4-aryl-2-haloimidazole-5-carboxylic ester of the general formula Ia or Ib

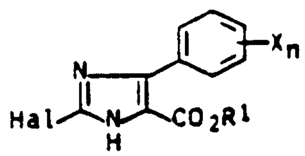

Ia

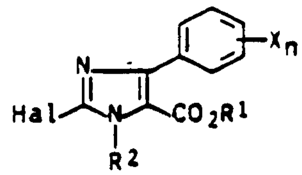

Ib

where

| | |
|---|---|
| $R^1$ | is $C_1$-$C_6$-alkyl, $C_3$-$C_6$-alkenyl or $C_3$-$C_6$-alkynyl, |
| $R^2$ | is $C_1$-$C_6$-alkyl, $C_3$-$C_6$-alkenyl or $C_3$-$C_6$-alkynyl, |
| Hal | is chlorine or bromine, |
| n | is 0 to 3, |
| X | is $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkylsulfinyl, $C_1$-$C_4$-alkylsulfonyl, $C_1$-$C_4$-haloalkylthio, cyano, nitro, carbo-$C_1$-$C_4$-alkoxy, N,N-di-$C_1$-$C_4$-alkyl-carbamoyl and/or halogen. |

2. A process for the preparation of a compound Ia as claimed in claim 1, which comprises subjecting a carbonyl compound II

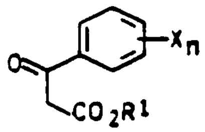

II

successively, in a conventional manner, without isolation of the intermediates, first to nitrosation with a nitrite III

$A^{\oplus}NO_2^{\ominus}$ III

where $A^{\oplus}$ is an alkali metal ion, in an aqueous acid to give IV

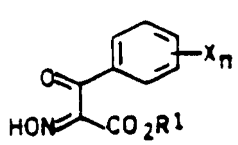

IV

then reduction to V

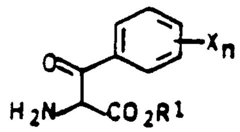

V

and conversion thereof directly with a cyanate VI

$B^{\oplus}OCN^{\ominus}$ VI

where $B^{\oplus}$ is an alkali metal cation or an ammonium cation, with the presence or absence of an inert organic solvent, into an imidazolone VII

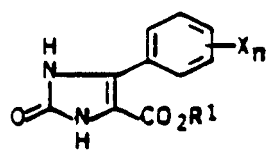

VII

which is then reacted with a halogenating agent to give Ia.

**3.** A process for the preparation of a compound Ib as claimed in claim 1, wherein a 4-aryl-2-haloimidazole-5-carboxylic ester Ia is reacted with an alkylating agent VIII

$R^2$-Y VIII,

where Y is halogen, sulfonate or sulfate, to give Ib.

**4.** A herbicide containing at least one 4-aryl-2-haloimidazole-5-carboxylic ester Ia and/or Ib as claimed in claim 1 and at least one herbicidal active ingredient from the group comprising cyclohexenone deriviatives of the formula X

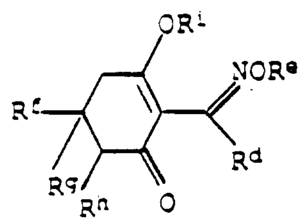

X

where

$R^d$ is $C_1$-$C_4$-alkyl;

$R^e$ is $C_1$-$C_4$-alkyl, $C_3$-$C_4$-alkenyl, $C_3$-$C_4$-alkynyl, $C_3$-$C_4$-haloalkenyl or thenylmethyl-(thienylmethyl) which can be substituted once by halogen;

$R^f$ is $C_1$-$C_4$-alkyl which can be substituted once by $C_1$-$C_4$-alkylthio or $C_1$-$C_4$-alkoxy;

a 5- or 6-membered saturated or monounsaturated ring system which, besides carbon members, can contain one oxygen, one sulfur or a sulfoxide or sulfone group, and which can carry up to three of the following: hydroxy, halogen, $C_1$-$C_4$-alkyl, $C_1$-$C_4$-haloalkyl, $C_1$-$C_4$-alkoxy and/or $C_1$-$C_4$-alkylthio;

a 10-membered saturated or monounsaturated heterocycle which contains two oxygens or sulfurs and can be substituted by up to three $C_1$-$C_4$-alkyl and/or methoxy groups;

phenyl, pyridyl or isoxazolyl, it being possible for these to carry up to three of the following:

$C_1$-$C_4$-alkyl, $C_1$-$C_4$-haloalkyl, $C_1$-$C_4$-alkoxy, $C_1$-$C_4$-alkylthio, $C_3$-$C_6$-alkenyloxy, $C_3$-$C_6$-alkynyloxy, $C_1$-$C_4$-alkoxy-$C_1$-$C_3$-alkyl, $C_1$-$C_4$-dialkoxy-$C_1$-$C_3$-alkyl, formyl, halogen and/or benzoylamino;

$R^g$ is hydrogen, hydroxy or, if $R^f$ is $C_1$-$C_6$-alkyl, a $C_1$-$C_6$-alkyl group;
$R^h$ is hydrogen, cyano, halogen or $C_1$-$C_4$-alkoxycarbonyl, and
$R^i$ is hydrogen or the equivalent of an environmentally compatible cation.

**5.** A herbicide as claimed in claim 4, containing a haloimidazole derivative Ia or Ib and a herbicide of the formula X, the Ia or Ib: X ratio being from 4:1 to 0.01:1 by weight.

**6.** A method for the selective control of unwanted plant growth, wherein a haloimidazole derivative of the formula Ia and/or Ib as claimed in claim 1 and a cyclohexenone derivative of the formula X as claimed in claim 4 are applied either simultaneously or successively before, during or after sowing of the crop plants, or before or during emergence of the crop plants.

**7.** A method for preventing damage to crop plants by herbicidal cyclohexenone derivatives of the formula X as claimed in claim 4, wherein the seed of the crop plants is treated with an antagonistic amount of a haloimidazole derivative of the formula Ia and/or Ib as claimed in claim 1.

**8.** A post-emergence method for the selective control of plant growth, wherein the leaves of the crop plants and the unwanted plants are treated either simultaneously or successively with a haloimidazole derivative of the formula Ia and/or Ib as claimed in claim 1 and with a cyclohexenone derivative of the formula X as claimed in claim 4.

**9.** A method as claimed in any of claims 6 to 8, wherein the crop plants are barley, wheat, corn, sorghum and rice.

## Revendications

**1.** Esters d'acides 4-aryl-2-halogénimidazol-5carboxyliques des formules genérales Ia et Ib

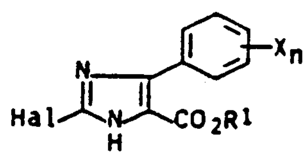

Ia

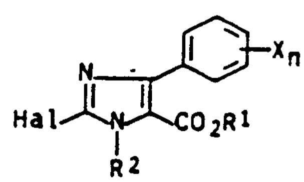

Ib

dans lesquelles les substituants et indices ont les significations suivantes :
$R^1$ = un groupe alkyle en C1-C6, un groupe alcényle en C3-C6 ou un groupe alcynyle en C3-C6,
$R^2$ = un groupe alkyle en C1-C6, un groupe alcényle en C3-C6 ou un groupe alcynyle en C3-C6,
Hal = chlore ou brome,
n = 0 à 3
X = un groupe alkyle en C1-C4, un groupe halogénalkyle en C1-C4, un groupe alcoxy en C1-C4, un groupe halogénalcoxy en C1-C4, un groupe alkylthio en C1-C4, un groupe alkylsulfinyle en C1-C4, un groupe alkylsulfonyle en C1-C4, un groupe halogénalkylthio en C1-C4, un groupe cyano, un groupe nitro, un groupe carbo-alcoxy en C1-C4, un groupe N,N-di-alkyl en C1-C4-carbamido et/ou un atome d'halogène.

**2.** Procédé de préparation des composés Ia selon la revendication 1, caractérisé par le fait que, successivement de manière en soi connue, sans isoler les stades intermédiaires, on nitrose d'abord un composé carbonylé II

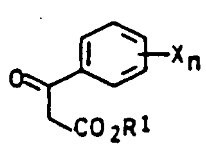

II

dans un acide aqueux, avec un nitrite III,

$A^{\oplus}NO_2^{\ominus}$ III

où $A^{\oplus}$ représente un ion métal alcalin, en obtenant IV

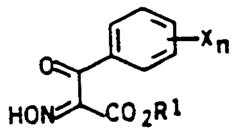

IV

ensuite on réduit IV en V

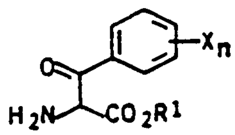

V

puis on transforme celui-ci directement, avec un cyanate VI,

$B^{\oplus}OCN^{\ominus}$ VI

où $B^{\oplus}$ est mis pour un cation métal alcalin ou un cation ammonium, en présence ou sans addition d'un solvant organique inerte, pour obtenir imidazole VII

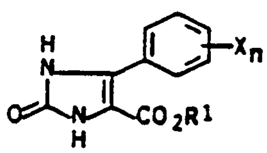

VII

que l'on met à réagir ensuite avec un agent d'halogénation pour obtenir Ia.

3. Procédé de préparation des composés Ib, selon la revendication 1, caractérisé par le fait que l'on met à réagir un ester d'acide 4-aryl-2-halogénimidazol-5-carboxylique Ia avec un agent d'alkylation VIII,

$R^2$-Y VIII

où Y représente halogène, un groupe sulfonate ou sulfate, pour obtenir Ib.

4. Agents herbicides contenant au moins un ester d'acide 4-aryl-2-halogénimidazol-5-carboxylique Ia et/ou Ib selon la revendication 1, ainsi qu'au moins un principe actif herbicide choirsi dans le groupe des dérivés de cyclohexénone, de formule X,

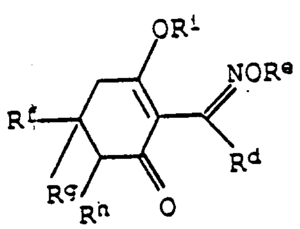

X

dans laquelle les substituants ont les significations suivantes :

$R^d$ = un groupe alkyle en C1-C4;
$R^e$ = un groupe alkyle en C1-C4, un groupe alcényle en C3-C4, un groupe alcynyle en C3-C4, un groupe halogénalcényle en C3-C4 ou un groupe thényle (=groupe thiénylméthyle) pouvant être substitué par un atome d'halogène;
$R^f$ = un groupe alkyle en C1-C4 pouvant être substitué une fois par alkyl-C1-C4-thio ou alcoxy en C1-C4;
un système cyclique à 5 ou 6 chaînons, saturé ou à une seule liaison insaturée, qui, outre des chaînons carbone, peut contenir un atome d'oxygène, de soufre ou un groupe sulfoxyde ou sulfone, ce cycle pouvant porter jusqu'à trois restes, à savoir hydroxy, halogène, alkyle en C1-C4, halogénalkyle en C1-C4, alcoxy en C1-C4 et/ou alkyl en C1-C4-thio;
un hétérocycle à dix chaînons, saturé ou à une seule liaison insaturée, qui contient deux atomes d'oxygène ou de soufre et peut être substitué par jusqu'à trois groupes alkyle en C1-C4 et/ou groupes méthoxy;
un groupe phényle, un groupe pyridyle ou un groupe isoxazole, ces groupes pouvant porte jusqu'à trois restes, à savoir : alkyle en C1-C4, halogénalkyle en C1-C4, alcoxy en C1-C4, alkyl en C1-C4-thio, alcényl en C3-C6-oxy, alcynyl en C3-C6-oxy, alcoxy en C1-C4-alkyle en C1-C3, dialcoxy en C1-C4-alkyle en C1-C3, formyle, halogène et/ou benzylamino;
$R^g$ = hydrogène, hydroxy ou, lorsque $R^f$ a la signification de groupe alkyle en C1-C6, un groupe alkyle en C1-C6;
$R^h$ = hydrogène, un groupe cyano, un atome d'halogène ou un groupe alcoxy en C1-C4-carbonyle et
$R^i$ = hydrogène ou l'équivalent d'un cation acceptable pour l'environnement.

**5.** Agents herbicides selon la revendication 4, contenant un dérivé d'halogénimidazole Ia ou Ib, ainsi qu'un herbicide de formule X, le rapport proportionnel de Ia ou Ib à X variant entre 4/1 et 0,01/1 parties en poids.

**6.** Procédé de lutte sélective contre la croissance de plantes indésirables, caractérisé par le fait que l'on répand, pendant ou après le semis des plantes cultivées, avant ou pendant la levée des plantes cultivées, simultanément ou successivement, un dérivé d'halogénimidazole de formule Ia et/ou Ib, selon la revendication 1, et un dérivé de cyclohexénone de formule X, selon la revendication 4.

**7.** Procédé pour empêcher un endommagement de plantes cultivées par des dérivés de cyclohexénone herbicides de formule X, selon la revendication 4, caractérisé par le fait que l'on traite les semences des plantes cultivées avec une quantité antagoniste efficace d'un dérivé d'halogénimidazole de formule Ia et/ou Ib, selon la revendication 1.

**8.** Procédé de lutte sélective contre la croissance de plantes indésirables, caractérisé par le fait que l'on traite les feuilles des plantes cultivées et des plantes indésirables, selon le procédé de post-émergence, simultanément ou successivement avev un dérivé d'halogénimidazole de formule Ia et/ou Ib selon la revendication 1 et avec un dérivé de cyclohexénone de formule X selon la revendication 4.

**9.** Procédé selon les revendications 6 à 8, caractérisé par le fait que les plantes de culture sont l'orge, le blé, le maïs, le sorgho et le riz.